# EUROPEAN PATENT APPLICATION

(11) **EP 1 418 525 A2**
(43) Date of publication of application: **12.05.2004**
(21) Application number: 03256914.7
(22) Date of filing: 31.10.2003
(51) Int. Cl.: G06F 19/00

(54) **Computer system and method for closed-loop support of patient self-testing**

(30) Priority: 31.10.2002 US 285406
(71) Applicant: Lifescan, Inc., Milpitas, California 95035-6312 (US)
(72) Inventor: Green, Michael R., Hopkinton NH 03229 (US); Knorr, Robert, Livermore CA 94550 (US); Cross, Suzanne, Kingsport TN 73664 (US); Kipp, Shari, San Jose CA 95120 (US); Earp, Brian, San Jose CA 95116 (US)
(74) Representative: Mercer, Christopher Paul

(57) **Abstract**

A computer system for the closed-loop support of patient self-testing includes data repository, healthcare provider interface, training interface, supplier, patient interface and analysis modules. The data repository module is configured to store patient enrollment and patient self-test information. The healthcare provider interface module is configured to receive patient enrollment and modified patient prescription information furnished by a healthcare provider and to transfer these to the data repository module. The healthcare provider interface module is also configured to communicate patient self-test result information to a healthcare provider. The training module is configured to receive patient diagnostic monitoring device training information and to transfer that information to the data repository module. The supplier module is configured to send notification information to a diagnostic monitoring device supplier, while the patient interface module is configured to receive patient self-test result information, transfer that information to the data repository module, and communicate modified patient prescription information to the patient. The analysis module is configured to validate patient's enrollment information; schedule patient training once the patient's enrollment information has been validated; evaluate patient diagnostic device training and patient self-test information stored in the data repository module; compile guideline compliance information based on patient enrollment, diagnostic device training and self-test information; and send notification information to the supplier module based on the evaluation. Also, a method for the closed-loop support of patient self-testing that includes scheduling of diagnostic monitoring device training based on validation of patient device training information.

## Description

### BACKGROUND OF THE INVENTION

### Field of the Invention

The present invention relates, in general, to computer systems and their related methods and, in particular, to computer systems and methods for the support of patient self-testing.

### Description of the Related Art

The management of medical conditions and their associated therapies in a non-clinical or otherwise remote setting can create a variety of support issues related to regulatory compliance documentation, logistics and patient convenience. For example, the management of medical conditions that require anticoagulation therapy frequently necessitates that patients adhere to a prescribed regimen of medication and diagnostic testing (e.g., diagnostic monitoring of prothrombin-time [PT] and/or International Normalization Ratio [INR]). **T**he results of the diagnostic testing can be used to adjust medication dosage and, thereby, prevent adverse events such as strokes or bleeding.

For medical conditions that require relatively frequent and routine diagnostic testing (e.g., on a weekly or daily basis), patient convenience suggests that the diagnostic testing be conducted as a patient self-test (PST) rather than solely in a clinical point-of-care (POC) environment. In this regard, the United States Food and Drug Admnistration (FDA) has approved various PT/INR diagnostic- monitoring devices for patient self-testing. Per the FDA, however, a prescription is required before such a diagnostic monitoring device can be made available to a patient. In addition, patients are required to complete a training program in the proper use of the diagnostic monitoring device prior to its being dispensed. These requirements create logistical support issues in that a diagnostic monitoring device cannot be dispensed to a patient without the healthcare professional or the entity distributing product to the patient verifying in some manner that the patient has a legitimate prescription and has been successfully trained.

In many medical management scenarios, the results of patient self-testing are provided on a regular basis to the patient's healthcare provider (e.g., treating physician). The healthcare provider then uses the patient self-test results to monitor the patient's status and determine whether modifications are needed to the patient's prescribed medication regimen. Since patient self-testing can conceivably occur at anytime and at any location (e.g., at the patient's home), support issues arise with regard to communicating patient self-test results to the healthcare provider, as well as communicating any modifications of a prescribed medication regimen back to the patient. In addition, the healthcare provider should be in a position to proactivety track a patient's self-test results in order to confirm that patient self-testing has occurred per the prescribed regime. In today's medical practice environment, this type of closed-loop support of patient self-testing can be an onerous and time-consuming task.

Financial reimbursement support issues can also arise during the management of medical conditions and their associated therapies. For example, Federal Regulation CFR §410.33, entitled "Independent Diagnostic Testing Facility (IDTF)," has established guidelines under which payments can be made when a medical management service is provided by an IDTF. One such guideline provides that services performed by an IDTF must be specifically ordered, in writing, by a patient's physician and the physician must use the results of the ordered service in management of a patient's medical care. Furthermore, financial reimbursement under Medicare and other governmental or private medical reimbursement entities can also be subject to guidelines. For example, proper documentation of clinical activity (eg., medication, diagnostic testing and adjustment of medication dosage) is often necessary to ensure physicians comply with regulatory requirements for Medicare billing. Therefore, insuring compliance with the various guidelines in order to secure reimbursement is yet another support issue that must be addressed during management of medical conditions and their associated therapies.

Still needed in the field, therefore, a system and method for the support of patient self-testing that provide a simple and expeditious solution to the logistical, closed-loop support, regulatory compliance and financial reimbursement requirements of patient self-testing.

### SUMMARY OF THE INVENTION

Computer systems and methods in accordance with the present invention provide for the closed-loop support of patient self-testing (eg., patient self-testing that includes PT/INR diagnostic monitoring) by automating, simplifying and expediting prescription and training validation, diagnostic monitoring device dispensation, closed-loop reporting and evaluation of self-test results and financial reimbursement activities. Use of computer systems and methods according to the present invention can, therefore, provide a complete, unified (integrated) best practices approach to the management of medical conditions and associated therapies that involve patient self-testing.

A computer system for the closed-loop support of patient self-testing according to an exemplary embodiment of the present invention includes a data repository module, a healthcare provider interface module, a traming interface module, a supplier module, a patient interface module and an analysis module. Alternatively, the computer system also includes a reimbursement interface module.

The data repository module of the computer system is configured to store patient enrollment information and patient self-test result information. The configuration of the healthcare provider interface module is such that the healthcare provider interface module can receive patient enrollment information and modified patient prescription information furnished by a healthcare provider and transfer these to the data repository module for storage. The healthcare provider interface module is also configured to communicate patient self-test result information to a healthcare provider.

The training module is configured to receive patient diagnostic monitoring device training information furnished by a training site and to transfer that information to the data repository module for storage. The supplier module is configured to send notification information to a diagnostic monitoring device supplier (e.g., a supplier of a PT/INR diagnostic monitoring device).

The patient interface module of the computer system is configured to receive patient self-test result information furnished by a patient for whom patient enrollment information has been received by the healthcare provider interface module. The patient interface module is also configured to transfer the patient self-test result information to the data repository module for storage, as well as to communicate modified patient prescription information received by the healthcare provider interface module to the patient.

The analysis module is configured to (i) validate a patient's enrollment information stored in the data repository module; (ii) schedule training for a patient once the patient's enrollment information have been validated; (iii) evaluate patient diagnostic device training information and patient self-test information stored in the data repository module; (iv) compile guideline compliance information based on patient enrollment information, patient diagnostic device training information and patient self-test information stored in the data repository module; and (v) send notification information to the supplier module based on the evaluation of patient diagnostic device training information, the notification information being sufficient to enable the provision of a diagnostic monitoring device to a patient by a diagnostic monitoring device supplier.

Since the computer system encompasses each of a data repository module, healthcare provider interface module, a training module, a supplier module, a patient interface module and analysis module, each configured as described above, the computer system is able to provide a total, integrated, closed-loop support of patient self-testing.

A process for the closed-loop support of patient self-testing according to an exemplary embodiment of the present invention includes storing patient enrollment information in a data repository module of a computer system. The patient enrollment information stored in the data repository module is, thereafter, validated. Subsequently, training in the use of a prescribed diagnostic monitoring device for a patient self-testing is scheduled. However, such scheduling only occurs for those patients whose enrollment information has been validated. The patient is then trained in the use of the diagnostic monitoring device and patient diagnostic monitoring device training information is stored in the data repository module of the computer system. Based on the patient diagnostic monitoring device training information, a diagaostic monitoring device is dispensed to the patient.

Once a patient has been dispensed a diagnostic monitoring device, the patient conducts a patient self-test(s) using the diagnostic monitoring device. The result of such a patient self-test(s) is a patient self-test result (e.g., a PT/INR value). Thereafter, the process includes storing the patient self-test result in the data repository module of the computer system, transferring the patient self-test results to a healthcare provider for evaluation and communicating, via the computer system as necessary, modified patient prescription information to the patient.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings of which:

FIG. 1 is a block diagram of a computer system for the closed-loop support of patient self-testing according to one exemplary embodiment of the present invention; and

FIG. 2 is a flow chart illustrating a sequence of steps in a process according to one exemplary embodiment of the present invention.

### DETAILED DESCRIPTION OF THE INVENTION

To be consistent throughout the present specification and for clear understanding of the present invention, the following explanation is hereby provided for a term used therein:

The term "patient prescription" can include a prescribed regimen of medication and diagnostic testing (eg.,, patient self-testing conducted using a prescribed diagnostic monitoring device), and/or physician prescribed algorithms for calculating medicine dosages, and/or contingent patient self-testing schedules, and/or a patient care plan identifying various patient self-test result alert levels (e.g. standard alert and emergency alert levels) or other prescription information useful in the management of a patient's medical condition and associated therapy.

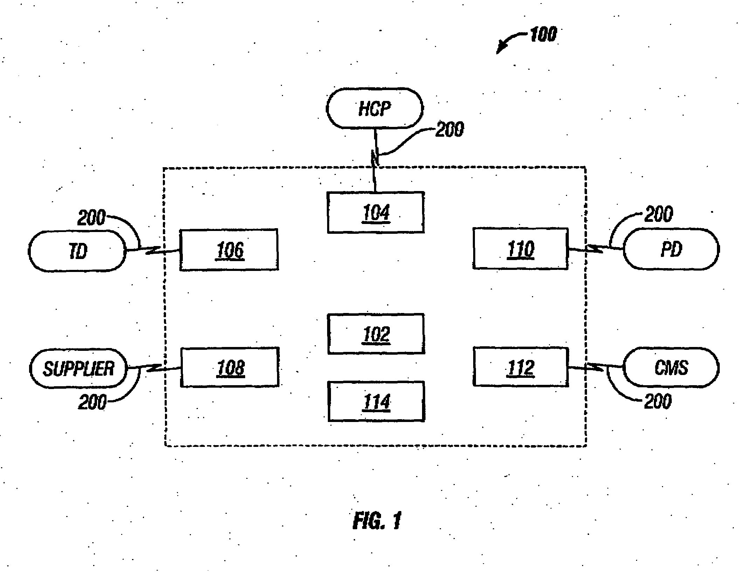

FIG. 1 is a block diagram of a computer system 100 (within the dashed lines) for the closed-loop support of patient self-testing according to one exemplary embodiment of the present invention. Computer system 100 includes a data repository module 102, a healthcare provider interface module 104, a training interface module 106, a supplier module 108, a patient interface module 110, a reimbursement interface module 112 and an analysis module 114. Modules 102,104,106,108,110,112 and 114 can be implemented using, for example, software resident on a server computer such that the modules are in operative communication with one another. Such a server computer can be any suitable server computer known in the art, including those commercially available from Sun Microsystems or IBM. Computer programming languages and web page protocols known to those skilled in the art, such as C, C++, HTML (''MyperText Markup Language''), Java, DHTML and JavaScript, can be used for such Implementation. It is recognized, however, that any suitable computer programming language or web page protocol can be used, including custom languages and protocols.

Alternatively, each or any combination of modules 102, 104, 106, 108, 110, 112 and 114 can be implemented on server computers or other hardware at separate locations and configured such that the modules are in operative communication with one another. In addition, it is contemplated that computer system 100 can be operated under the control of an IDTF or other similar entity, thus relieving healthcare providers of the burden of maintaining the computer system.

Data repository module 102 is configured for storing patient enrollment information and patient self-test result information. The patient enrollment information stored in data repository module 102 can include, for example, the following items:
- physician's (or other healthcare provider's) name, address, state license number, system usemame, system password, phone number and facsimile number;
- patient information, such as patient's name, system username, system password, mailing address, e-mail address, phone number, facsimile number and billing option (e.g., IDTF billing, self-billing or patient out-of-pocket); and
- a patient prescription.

The patient -self-test result information stored in data repository module 102 can include, for example, the result of a diagnostic monitoring test conducted by the patient (e.g., a FT/INR diagnostic monitoring test result or blood glucose test result), patient self-test ancillary information (e.g., the date and time of a particular patient self-test), patient responses to standardized questions and other patient provided medical information. Furthermore, data repository module 102 can be optionally configured such that each patient is associated with the appropriate healthcare provider (e.g., a physician who is responsible for the management of that patient's medical condition and associated therapy).

Data repository module 102 can be implemented, for example, as a relational database or any other suitable database known to one skilled in the art including, but not limited to, an Oracle relational database.

Healthcare provider interface module 104 is configured to perform, for example, the following functions:
- receive patient enrollment information furnished by healthcare providers (e.g., physicians, pharmacists, or other medically and legally qualified healthcare provider);
- transfer the patient enrollment information to the data repository module for storage;
- communicate patient self- test result information to healthcare providers; and
- receive modified patient prescriptions or other therapeutic information (e.g, instructions to avoid certain foods) from healthcare providers and transfer the modified patient prescriptions to the data repository module for storage.

Furthermore, if desired, heatthcare provider interface module 104 and/or reimbursement interface module 112 (described in detail below) can be configured to provide Health Plan Employer Data and Information Set (HEDIS) reports to a healthcare provider or reimbursement entity, respectively/In such a circumstance, computer system 100 would be configured to determine, for example, the frequency of patient self-testing on a patient-by-patient basis (e.g., all patients under a given physicians care). Such frequencies can then be compared to local or national patient self-testing frequencies, correlated to adverse events, and/of correlated to the duration a patient is in a therapeutic medication range. These comparisons can then serve as aids in measuring care effectiveness and establishing a best practices benchmark.

Healthcare provider interface module 104 can receive the patient enrollment information and modified patient prescriptions and communicate patient self-test result information via any known data receipt/communication method inducing, but not limited to, interaction via voice transmission, touchtone-based entry, keyboard-based entry, wireless transmission, and/of direct download from a peripheral device. Furthermore, the communication of patient self-test result information can also be accomplished via fax, e-mail, pager or standard mail techniques under the control of the healtcare provider interface module 104.

Receipt of the patient enrollment information and modified patient prescriptions and communication of patient self-test result information via a wide area network, for example a public network (e.g., the Internet), is particularly beneficial in terms of providing an automated and expeditious means for receiving and communicating information. In addition, the use of a distributed communication network, such as broadband Internet is a relatively non-cumbersome and inexpensive communication medium. Such wide area and distributed communication networks can be any type of suitable network including a public network, a private network, the Internet (i.e., a global internetwork or networks), an Intranet, an Extranet, a virtual private network (VPN), a non-TCP/IP network or a wireless network.

In the embodiment of FIG 1., healthcare provider interface module 104 is shown as linked to a healthcare provider device (HCP) via Internet 200. The HCP device can be any suitable Intemet-enabled device capable of communicating via the Internet, including communication via voice and the display of images. Such an Internet-enabled device can be, for example, an internet-enabled personal digital assistant (PDA), an internet-enabled personal computer (PC), an internet-enabled cellular phone, or an interactive television that is employed by a healthcare provider for interacting with computer system 100. One skilled in the art wilt recognize that although a single HCP device is illustrated in FIG. 1, computer systems according to the present invention can be configured to operate with a plurality of HCP devices.

Training interface module 106 is configured to receive patient diagnostic monitoring device training information furnished by a training site and to the transfer patient diagnostic device training information to the data repository module 102 for storage. The patient diagnostic monitoring device training information can include, for example, patient name and/or other identifying information (e.g., social security number), a patient's training status (e.g., untrained, failed training or passed training), and patient training date. In the embodiment of FIG. 1, healthcare provider interface module 106 is shown as linked to an internet-enabled training device (TD) via Internet 200. However, training interface module 106 can receive the patient diagnostic monitoring device training information via any known data receipt/conmunication method including, but not limited to, interaction via voice transmission, touchtone-based entry, keyboard-based entry, and/or direct download from a peripheral device.

The internet-enabled training device (TD) can comprise, for example, a kiosk with a self-contained computer, a touchscreen and/or display device for interaction with a patient. In such a kiosk, software resident on the computer can be configured to, for example, enable identification of a patient, a patient's trainer and a diagnostic monitoring device for which the patient has undergone or is undergoing training. If desired, upon completion of a patient's training, an exam can be administered to a patient via the computer, touchscreen or other display device to measure the effectiveness of the training. The exam results can then be communicated to training interface module 106 as patient diagnostic monitoring device training information.

Supplier module 108 is configured to send (eg., electronically transfer) notification information to a diagnostic monitoring device supplier (referred to simply as "Supplier" in FIG. 1) via any suitable means, such as the Internet 200. It is contemplated that such notification information will be used by a diagnostic monitoring device supplier for delivery (i.e., dispensing) of a prescribed diagnostic monitoring device to a patient. Such diagnostic monitoring devices include, but are not limited to, PT/INR kits for the quantitative determination of Prothrombin time (PT) and/or INR in capillary and venous whole blood as an aid in monitoring Warfarin therapy by laypersons in the home for the purposes of patient self testing (PST). Such kits can include, for example, a meter, disposable reagent test strip and lancet(s).

Patient interface module 110 of computer system 100 is configured to perform the following functions in support of patient self-testing:
- receive patient self-test result information furnished by a patient for whom patient enrollment information has been received by the healthcare provider interface module;
- transfer the patient self-test result information to the data repository module for storage; and
- communicate updated patient treatment instructions received by the healthcare provider interface module to the patient.

In the embodiment of FIG. 1, patient interface module 110 is shown as linked to an internet-enabled patient device (PD) via Internet 200. The internet-enabled patient device can be, for example, integrated into the diagnostic medical device used by the patient for patient self-testing by including a serial data port or other suitable hardware and software within the diagnostic medical device. Alternatively, patient interface module 110 can receive the patient diagnostic monitoring device training information via any known data receipt/communication method including, but not limited to, interaction via voice transmission, touchtone-based entry, keyboard-based entry, direct download from a peripheral device, and/or wireless transmission.

Reimbursement interface module 112 is configured to store and retrieve guideline compliance information from data repository module 102 and transfer the guideline compliance information to a reimbursement entity (e.g., the Centers for Medicaid and Medicare Services [CMS] or a private insurance entity) to facilitate the provision of fees to various entities involved in the management of an enrolled patient's medical condition and associated therapy. Such fees can include, for example, fees due to healthcare providers or an IDTF based on guideline compliant services rendered. Reimbursement interface module 112 can transfer the guidance compliance information using any suitable transfer means including electronic means, such as via the Internet.

In the embodiment of FIG. 1, reimbursement interface module 112 is shown as linked to the CMS via Internet 200. Alternatively, reimbursement interface module 112 can transfer the guidance compliance information via any known data receipt/communication method including, but not limited to, interaction via voice transmission, touchtone-based entry, keyboard-based entry, direct download from a peripheral device, standard mail, e-mail, and/or wireless transmission.

Analysis module 114 of computer system 100 is adapted to perform the allowing functions in support of patient self-testing:
- validation of a patient's enrollment information stored in the data repository module (for example, validation of a patient prescription by assessing whether a physician's signature is on the prescription and whether the physician has a valid medical license);
- scheduling of training for a particular patient once the particular patient's enrollment information have been validated;
- evaluation of patient diagnostic device training information stored in the data repository module (e.g., validating that a particular patient has successfully completed training by, for example, assessing whether an appropriate healthcare professional has signed a patient training verification document);
- evaluation of patient self-test information stored in the data repository module; and
- compilation, of guideline compliance information based on patient enrollment information, patient diagnostic device training information and patient self-test information stored in the data repository module; and
- sending of notification information to the diagnostic monitoring device supplier module based on the evaluation of patient diagnostic monitoring device training information, the notification information being sufficient to enable the provision of a diagnostic monitoring device to a patient by a diagnostic monitoring device supplier.

In the circumstance that patient self-testing requires a diagnostic monitoring device that utilizes a consumable (e.g., a disposable reagent test strip), analysis module 114 and supplier module 108 can be optionally configured to send notification information sufficient to enable the provision (dispensing) of such consumables to a patient.

Furthermore, if desired, analysis module 114 can be configured to perform a variety of information management functions including tracking prescription information history and medication interactions/interferences for each enrolled patient. Computer system 100 can also be configured to immediately alert a healthcare provider when a patient's self-test result information is not in compliance with relevant patient self-test alert levels.

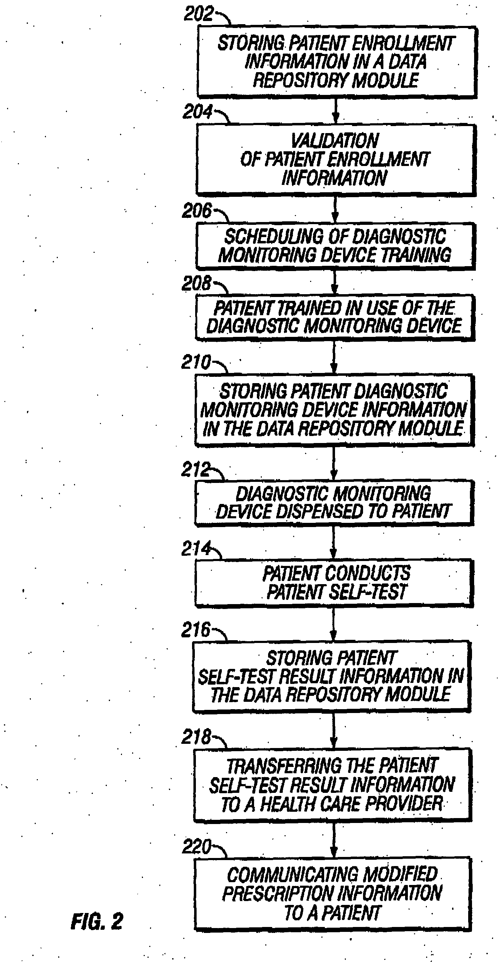

FIG. 2 is a flow chart illustrating a sequence of steps in a process 300 for the closed-loop support of patient self-testing according to one exemplary embodiment of the present invention. First, at step 202, patient enrollment information is stored in a data repository module of a computer system. Next, the patient enrollment information stored in the data repository module is validated, as set forth in step 204. This validation can include, for example, validation of a prescribing physician's, license and otherwise reviewing the patient enrollment information for errors.

Next, training in the use of a prescribed diagnostic monitoring device for a patient self-testing is scheduled, as set forth in step 206. Such scheduling only occurs for those patients whose enrollment information has been validated. If desired, the scheduling can occur automatically upon validation of patient enrollment information and such scheduling can be automatically communicated to the patient and a training site.

The patient is then trained in the use of the diagnostic monitoring device, as set forth in step 208, followed by storing of patient diagnostic monitoring device training information in the data repository module of the computer system, as set forth in step 210. Based on the patient diagnostic monitoring device training information, a diagnostic monitoring device is dispensed to the patient, as reflected in step 212. For example, diagnostic monitoring devices will only be dispensed to patients, who have successfully completed the diagnostic monitoring device training as indicated by the diagnostic monitoring device training information.

Once a patient has been dispensed a diagnostic monitoring device, the patient conducts a patient self-test using the diagnostic monitoring device, as set forth in step 214. The result of such a patient self-test is patient self-test result information (e.g., a PT/INR value and patient self-testing ancillary information).

As illustrated in FIG. 2, process 300 also includes the steps of storing the patient self-test result information in the data repository module of the computer system (step 216), transferring the, patient self-test result information to a healtheare provider for evaluation (step 218) and communicating, via the computer system, modified patient prescription and, optionally, other therapeutic information to the patient (step 220).

Once apprised of the present disclosure, one skilled in the art will recognize that process 300 can be implemented using computer System 100 described above and that processes according to the present invention can be adapted to encompass any of the functions described above with respect to computer system 100. For example, any of the scheduling/storing/communicating steps can be accomplished via the Internet.

Optionally, process 300 can include additional steps to compile guideline compliance information in the data repository module and transfer the guideline compliance information to a reimbursement entity (e.g., the CMS).

Computer systems and methods according to the present invention can be efficiently implemented via software and hardware, for example a modular C/C++ programming language library on an operating system with an in-memory database. Moreover, one skilled in the art will recognize that other programming languages (e.g., Java, Perl, Visual Basic and Pascal) and a variety of operating systems (eg., Windows, Solaris and Linux) can be utilized to implement the present invention.

Exemplary embodiments of the present invention have been described with respect to patient self-testing related to anticoagulant therapy (i.e., PT/INR monitoring). However, one skilled in the art will recognize that computer systems and methods according to the present invention can be utilized in support of a wide variety of patient self-testing including, but not limited to, PT/INR self-testing and blood glucose patient self-testing.

It should be understood that various alternatives to the embodiments of the invention described herein may be employed in practicing the invention. It is intended that the following claims define the scope of the invention and that structures and methods within the scope of these claims and their equivalents be covered thereby.

## Claims

1. A computer system for the closed-loop support of patient self-testing, the computer system comprising:
a data repository module for storing patient enrollment information and patient. self-test result information;
a healthcare provider interface module configured to:
receive patient enrollment information furnished by a healthcare provider;
transfer the patient enrollment information to the data repository module for storage;
communicate patient self-test result information to a healthcare provider; and
receive modified patient prescription information from a healthcare provider and transfer the modified patient prescription information to the data repository module for storage;
a training module configured to:
receive patient diagnostic monitoring device training information furnished by a training site; and
transfer patient diagnostic device training information to the data repository module for storage;
a supplier module configured to send notification information to a diagnostic monitoring device supplier;
a patient interface module configured to:
receive patient self-test result information furnished by a patient for whom patient enrollment information has been received by the healthcare provider interface module;
transfer the patient self-test result information to the data repository module for storage; and
communicate modified patient prescription information received by the healthcare provider interface module to the patient; and
an analysis module configured to:
validate a patient's enrollment information stored in the data repository module;
schedule training for a patient once the patient's enrollment information have been validated;
evaluate patient diagnostic device training information and patient self-test information stored in the data repository module;
compile guideline compliance information based on patient enrollment information, patient diagnostic device training information and patient self-test information stored in the data repository module; and
send notification information to the supplier module based on the evaluation of patient diagnostic device training information, the notification information being sufficient to enable the provision of a diagnostic monitoring device to a patient by a diagnostic monitoring device supplier.

2. The computer system of claim 1 further comprising:
a reimbursement module configured to:
retrieve guideline compliance information from data repository module; and
transfer the guideline compliance information to a reimbursement entity.

3. The computer system of claim 2, wherein the data repository module, healthcare provider interface module, training interlace module, supplier module, analysis module and reimbursement module are resident on a server computer.

4. The computer system of claim 1, wherein the healthcare provider interface module is configured to receive at least one of the patient enrollment information and patient prescription information via the Internet.

5. The computer system of claim 1, wherein the patient interface module is configured to receive patient self-test result information and communicate modified patient prescription information via the Internet.

6. The computer system of claim 1, wherein the supplier module is configured to send notification information to a diagnostic monitoring device supplier via the Internet.

7. The computer system of claim 2, wherein the reimbursement module is configured to transfer the guideline compliance information to a reimbursement entity via the Internet.

8. The computer system of claim 1, wherein the healthcare provider interface module is configured to communicate patient self-test result information to a healthcare provider that has been associated with the patient who furnished the patient self-test information.

9. The computer system of claim 1, wherein the analysis module and supplier module are configured to send notification information sufficient to enable the provision of consumables to a patient.

10. The computer system of claim 1, wherein:
the healthcare provider interface module is further configured to receive therapeutic information from the healthcare provider and transfer the therapeutic information to the data repository module for storage; and
the patient interface module is further configured to communicate the therapeutic information received by the healthcare provider module to the patient

11. The computer system of claim 10, wherein the patient interface module is further configured to communicate modified patient prescription information and therapeutic information via the Internet.

12. A method for the closed loop support of patient self-testing, the method comprising:
storing, in a data repository module of a computer system, patient enrollment information;
validating the stored patient enrollment information stored in the data repository module;
scheduling training for a patient whose patient enrollment information has been validated;
training of the patient in the use of a diagnostic monitoring device;
storing of patient diagnostic monitoring device training information in the data repository module of the computer system;
dispensing of a diagnostic monitoring device to the patient based on the patient diagnostic monitoring device training information stored in the data repository module;
conducting, by the patient, a patient self-test using the diagnostic monitoring device, thereby producing patient self-test result information;
storing the patient self-test result information in the data repository module of the computer system;
transferring the patient self test result information to a healtheare provider for evaluation;
communicating, via the computer system, modified patient prescription information to the patient.

13. The method of claim 12, further including the steps of:
compiling guideline compliance information in the data repository module; and
transferring the guideline compliance information to a reimbursement entity.

14. The method of claim 12, wherein the scheduling step occurs automatically upon validation of the stored patient enrollment information.

15. The method of claim 12, wherein at least one of the scheduling step, storing steps and communicating steps are accomplished via the Internet.

16. The method of claim 12, wherein the dispensing step includes dispensing a PT/INR diagnostic monitoring device to the patient.

17. The method of claim 12, wherein the transferring step includes the patient self-test result information to a healthcare provider for documentation and the communicating step includes communicating therapeutic information to the patient.
